Europäisches Patentamt

European Patent Office

Office européen des brevets

(19)

(11) Veröffentlichungsnummer: **0 230 570**
**B1**

(12) **EUROPÄISCHE PATENTSCHRIFT**

(51) Int. Cl.⁴: **A61L 27/00, A61K 6/06**

(45) Veröffentlichungstag der Patentschrift:
28.03.90

(21) Anmeldenummer: 86116817.7

(22) Anmeldetag: 03.12.86

(54) **Poröses Hydroxylapatit-Material.**

(30) Priorität: 03.12.85 DE 3542744

(43) Veröffentlichungstag der Anmeldung:
05.08.87 Patentblatt 87/32

(45) Bekanntmachung des Hinweises auf die Patenterteilung:
28.03.90 Patentblatt 90/13

(84) Benannte Vertragsstaaten:
AT BE CH DE ES FR GB IT LI NL SE

(56) Entgegenhaltungen:
EP-A- 0 022 724
CH-A- 643 732
FR-A- 2 223 325
FR-A- 2 350 826
FR-A- 2 460 710

CHEMICAL ABSTRACTS, Band 83, Nr. 21, 24.
November 1975, Seite 428, Zusammenfassung
Nr. 177172x, Columbus, Ohio, US; G. BLUNDEN et al.:
"Commercial collection and utilization of maerl", &
ECON. BOT. 1975, 29(2), 140-5

Die Akte enthält technische Angaben, die nach dem
Eingang der Anmeldung eingereicht wurden und die
nicht in dieser Patentschrift enthalten sind.

(73) Patentinhaber: Ewers, Rolf, Prof. Dr. Dr.,
Graf-Spee-Strasse 46, D-2300 Kiel 1(DE)

(72) Erfinder: Ewers, Rolf, Prof. Dr. Dr., Graf-Spee-Strasse 46,
D-2300 Kiel 1(DE)
Erfinder: Kasperk, Christian, Dr. Dr., Lorenzendamm 20,
D-2300 Kiel 1(DE)

(74) Vertreter: Weber, Otto Ernst, Dipl.-Phys.,
Hofbrunnstrasse 36, D-8000 München 71(DE)

## Beschreibung

Die Erfindung betrifft ein poröses Hydroxylapatit-Material und dessen Verwendung in der Medizin, Zahnmedizin und Tiermedizin.

Aus der US-Patentschrift 3 929 971 ist bereits ein synthetisches, poröses Hydroxylapatit-Material bekannt, welches eine spezielle Mikrostruktur aufweist und als Biomaterial, z.B. als Prothetikmaterial, Knochenimplantate und dergleichen verwendet werden kann. Dieses vorbekannte Hydroxylapatit-Material wird aus den Karbonatskeletten von Meerestieren, insbesondere Korallen und Seesternen hergestellt.

**Aufgabe** der vorliegenden Erfindung ist die Bereitstellung eines weiteren porösen Hydroxylapatit-Materials, das aufgrund der bei seiner Herstellung verwendeten Ausgangsmaterialien eine spezielle Porosität aufweist und in spezieller Form vorliegt.

Zur Lösung dieser Aufgabe dient das poröse HydroxylapatitMaterial, das dadurch gekennzeichnet ist, daß das Material aus dem in Hydroxylapatit umgewandelten kalziumreichen Grundskelett von kalkinkrustierenden Algen hergestellt ist.

Gemäß bevorzugten Ausführungsformen sind die Poren des porösen Hydroxylapatit-Materials durch ein resorbierbares oder ein knocheninduktives Material wie Knochengelatine ausgefüllt.

Das erfindungsgemäße poröse Hydroxylapatit-Material findet insbesondere in der Herstellung von Knochenersatz, Zahnwurzelersatz, Stabilisierungsmaterial, Hohlkörperersatz, Augmentationsmaterial, Unterfütterungsmaterial oder Defektfüllungsmaterial.

Zur Herstellung des Hydroxylapatit-Materials können gemäß der Erfindung sämtliche Algen, insbesondere des Eu- und Sublitorals der Weltmeere, insbesondere Spezies der Corallinaceae wie Corallina officinalis, Corallina rubens, Lithothamnion calcarum und Spezies der Codiaceae, z.B. Halimeda spec., einschließlich benthisch-krustenförmiger Algen wie Lithothamnion spec. und Lithophyllum spec. verwendet werden, die überhaupt Kalk inkrustieren, sei es auch nur geringfügig.

Um das Kalkskelett der Algen zu erhalten, müssen die organischen Bestandteile der Algen zunächst entfernt werden. Dies kann durch sogenannte "Mazeration" erfolgen. Hierzu kann beispielsweise vitales oder getrocknetes Algenmaterial durch Kupferkomplexsalze, z.B. Schweizer's Reagens, oder mit Lithiumchlorid oder-bromidlösungen behandelt werden, oder es kann eine Pyrolyse durchgeführt werden. Solche Mazerationsverfahren, d.h. Arbeitsweisen zur Entfernung des organischen Materials, sind ebenfalls in der zuvor genannten US-PS 3 929 971 beschrieben, z.B. das Eintauchen in verdünnte Natriumhypochloritlösungen.

Nach der Mazeration mittels Schweizer's Reagens oder Lithiumchloridlösung oder nach einer Pyrolyse wird das von organischer Substanz restlos befreite Kalziumcarbonatgrundmaterial, das in Form eines Granultates vorliegt, gründlich mit destilliertem Wasser gewaschen. Die Korngröße des Materials liegt zwischen 0,1 und 2 mm.

Das aus den Algen gewonnene, kalziumreiche Grundskelett wird dann in an sich bekannter Weise in Hydroxylapatit umgewandelt. Diese Umwandlung in Hydroxyalapatit erfolgt üblicherweise nach einem hydrothermischen Verfahren unter Anwendung eines Phosphatsalzbades. Hierzu können Alkalimetallphosphate wie Natriumorthophosphat, Kaliumorthophosphat und Ammoniumorthophosphat, saure Phosphate und Mischphosphate verwendet werden. Ebenfalls kann Orthophosphorsäure hierzu verwendet werden. Diese hydrothermale Behandlung zur Umwandlung von Kalziumcarbonat in Hydroxylapatit ist ebenfalls in der zuvor genannten US-Patentschrift 3 929 971 beschrieben. Die hydrothermale Behandlung wird in üblicher Weise bei erhöhtem Druck und erhöhter Temperatur durchgeführt.

Ein Beispiel für eine solche Behandlung besteht darin, die porösen, mazerierten Granula aus Kalk inkrustierenden Algen mit Natriumhydrogenphosphat und bidestilliertem Wasser in einem Verhältnis 1 : 1 : 4 in einem geschlossenen Behälter für 2 bis 3 Tage unter folgen Bedingungen zu halten:

    a) 300 °C und 500 bar oder
    b) 600 °C und 1000 bar.

Je nach den angewandten physikalischen Bedingungen kann auf die Resorbierbarkeit des Materials in der Knochenchirurgie Einfluß genommen werden.

Nach der hydrothermalen Behandlung liegt das Hydroxylapatit-Material üblicherweise in Granulatform vor. Aus dieser Granulatform können kompakte Stücke durch Agglomeration, z.B. durch übliche Sinterverfahren, gegebenenfalls unter Anwendung von Druck und unter Zusatz üblicher Sinterhilfsmittel hergestellt werden.

Zur Agglomeration des Granulats zu kompakten Stücken werden diese zunächst zu Formkörpern gepreßt und diese dann nach einer etwa vierstündigen Vorwärmphase bei einer Temperatur von 1000 bis 1400°C während 4 bis 5 Stunden gebrannt, anschließend wird z.B. während 4 bis 5 Stunden abgekühlt. Zur bewußten Beeinflussung der Porengröße des Hydroxylapatitkörpers nach Sinterung wird die Sinterung unter Zugabe empirisch ermittelter Mengen $H_2O$ (bidest.) durchgeführt.

Die Poren des erfindungsgemäßen Hydroxylapatit-Materials können mit resorbierbaren Materialien gefüllt sein. Hierdurch kann die Verträglichkeit des Hydroxylapatit-Materials bei der Verwendung in der Medizin, Zahnmedizin oder Tiermedizin verbessert werden, insbesondere kann die bei porösem Hydroxylapatit-Material auftretende Infektionsrate durch Füllung der Poren gesenkt und/oder die Knocheninduktion gesteigert werden. Als resorbierbare Materialien können lyophilisiertes heterologes Collagenvlies, homologes lyophilisiertes Fibrin, Gips, Proteine wie Gelatine oder Polymilchsäurepräparate, z.B. Polydyoxanon verwendet werden.

Ein Vorteil einer Ausführungsform des erfindungsgemäßen Hydroxylapatit-Materials im Vergleich zu porösem Hydroxylapatit-Material, das aus Korallenmaterialien gemäß der US-PS 3 929 971 hergestellt wurde, liegt in der Granulatform, die bei bestimmten Anwendungsmöglichkeiten Vorteile bietet.

**Patentansprüche**

1. Poröses Hydroxylapatit-Material, dadurch **gekennzeichnet**, daß das Material aus dem in Hydoxylapatit umgewandelten kalziumreichen Grundskelett von kalkinkrustierenden Algen hergestellt ist.

2. Poröses Hydroxylapatit-Material nach Anspruch 1, dadurch **gekennzeichnet**, daß seine Poren durch ein resorbierbares Material ausgefüllt sind.

3. Poröses Hydroxylapatit-Material nach Anspruch 1, dadurch **gekennzeichnet**, daß seine Poren durch ein knocheninduktives Material ausgefüllt sind.

4. Poröses Hydroxylapatit-Material nach Anspruch 3, dadurch **gekennzeichnet**, daß seine Poren durch Knochengelatine ausgefüllt sind.

5. Verwendung des porösen Hydroxylapatit-Materials nach einem der Ansprüche 1 bis 4 zur Herstellung von Knochenersatz, Zahnwurzelersatz, Augmentationsmaterial, Stabilisierungsmaterial, Hohlkörperersatz oder Defektfüllungsmaterial.

**Claims**

1. A porous hydroxyl apatite material, characterized in that the material is made from the calcium-rich basic skeletons of lime-encrusting algae converted into hydroxyl apatite.

2. A porous hydroxyl apatite material according to claim 1, characterized in that its pores are filled with a re-absorbable material.

3. A porous hydroxyl apatite material according to claim 1, characterized in that its pores are filled with a bone-inducing material.

4. A porous hydroxyl apatite material according to claim 3, characterized in that its pores are filled with bone marrow.

5. Use of the porous hydroxyl apatite material according to one of claims 1 to 4 for preparation of artificial bone, artificial tooth root, augmentation material, stabilization material, artificial hollow bodies or defect-filling material.

**Revendications**

1. Matériau poreux à base d'hydroxylapatite, caractérisé en ce que le matériau est obtenu à partir du squelette de base riche en calcium, converti en hydroxylapatite, d'algues fixant le calcaire.

2. Matériau poreux à base d'hydroxylapatite selon la revendication 1, caractérisé en ce que ses pores sont remplis d'un matériau résorbable.

3. Matériau poreux à base d'hydroxylapatite selon la revendication 1, caractérisé en ce que ses pores sont remplis d'un matériau ostéoformateur.

4. Matériau poreux à base d'hydroxylapatite selon la revendication 3, caractérisé en ce que ses pores sont remplis de gélatine d'os.

5. Utilisation du matériau poreux à base d'hydroxylapatite selon l'une quelconque des revendications 1 à 4, pour la fabrication de substitut d'os, substitut de racines dentaires, matériau de complément, matériau de stabilisation, matériau de remplacement de corps creux ou matériau de comblement de lacunes.